# EUROPÄISCHE PATENTANMELDUNG

(11) **EP 2 789 325 A1**
(43) Veröffentlichungstag der Anmeldung: **15.10.2014**
(21) Anmeldenummer: 14000767.5
(22) Anmeldetag: 04.03.2014
(51) Int. Cl.: A61H 33/06, A61N 5/06, H05B 3/00

(54) **IR-Strahler mit Doppelverglasung**

(30) Priorität: 11.04.2013 AT 2822013; 30.04.2013 EP 13002288
(71) Anmelder: abatec group AG, 4844 Regau (AT)
(72) Erfinder: Niederndorfer, Friedrich, 4863 Seewalchen am Attersee (AT)

(57) **Zusammenfassung**

Die Erfindung betrifft einen Infrarotstrahler für die Beheizung einer Wellnesseinrichtung, welcher eine in einem Gehäuse (1) angeordnete Infrarotstrahlungsquelle (3) aufweist, sowie eine Abdeckung aus lichtdurchlässigem Material.

Die Abdeckung ist durch zwei in einem Abstand zueinander angeordnete Scheiben (4, 5) gebildet, wobei das Material der der Infrarotstrahlungsquelle (3) näher liegenden Scheibe (4) einen höherem Absorptionsgrad für die von der Infrarotstrahlungsquelle (3) ausgesandte Strahlung aufweist als das Material der von der Infrarotstrahlungsquelle (3) weiter entfernt liegenden Scheibe (5).

## Beschreibung

Die Erfindung betrifft einen als Beheizung in einer beheizten Wellnesseinrichtung anzuwendenden Infrarotstrahler, sowie eine dementsprechend ausgestattete beheizte Wellnesseinrichtung. Unter dem Begriff "Beheizte Wellnesseinrichtung" ist in dieser Schrift eine Infrarotkammer oder eine Saunakammer zu verstehen, in welcher bestimmungsgemäß Menschen Platz nehmen und dort an Luft einer gegenüber äußerer Umgebungstemperatur erhöhten Temperatur ausgesetzt werden.

Sehr ungefährliche aber doch oftmals wohltuende Infrarotstrahlung kann durch Erwärmung sehr großer Flächen auf relativ niedrige Temperaturen (beispielsweise 60°C) hervorgerufen werden. Infrarotstrahlung wird dabei ausschließlich im "fernen IR-Spektralbereich" (langwelliger, vom sichtbaren Spektralbereich weit entfernter Spektralbereich) abgestrahlt. Gegenüber Infrarotstrahlung, welche auch deutlich kurzwelligere Anteile in nennenswerter Intensität enthält, ist nachteilig, dass die Wirkung nur langsam fühlbar wird, dass die Eindringtiefe in bzw. unter die Haut sehr niedrig ist und dass wegen der erforderlichen Großflächigkeit Platzprobleme entstehen können.

Intensivere, schnellere und gesundheitlich breitere Wirkung wird mit Infrarotstrahlern erzielt, welche auch im mittleren und nahen IR-Spektralbereich strahlen. Dafür geeignete Strahler operieren mit kleinflächigeren, dafür aber sehr heißen Strahlungsquellen, welche pro Oberfläche relativ hohe Strahlungsleistung emittieren. Neben IR-Strahlung emittieren diese Strahler auch Strahlung im sichtbaren Spektralbereich (vorwiegend mit überwiegend rotem Farbeindruck) und oft auch nennenswert Strahlung im nahen UV-Spektralbereich. Diese zusätzlichen Spektralbereiche sind zumeist erwünscht, typischerweise weil damit Farbe und Wirkung des Lichtes den diesbezüglich vorteilhaften Ausprägungen des Sonnenlichtes ähnlicher werden. Dabei kommt es allerdings auch zu Gefahren. Vor allem Licht im nahen Infrarotbereich kann in den Augen zu Schäden führen, da es zwar wie sichtbares Licht in durch die Linse des Auges fokussiertem Zustand an die Netzhaut des Auges geleitet wird, aber nicht wie sichtbares Licht im Fall von zu hoher Intensität einen Schutzreflex des Auges (Blinzeln) auslöst. Wegen der extrem hohen Temperatur der Strahlungsquelle sind auch Maßnahmen erforderlich um zu verhindern, dass durch diese Hitze Teile entzündet oder anderweitig zerstört werden oder Menschen zu Schaden kommen können.

Die Schriften DE 2313103 A1, DE 10254277 A1, DE 196 28 494 A1, DE 199 42 632 A1 und EP 2 168 632 A2 zeigen Infrarotstrahler für beheizte Wellnesseinrichtungen. Sofern konzentrierte und damit sehr heiße Infrarotstrahlungsquellen verwendet werden, sind die Strahlungsquellen durch Schutzgitter abgedeckt, damit sie nicht berührt werden können. Abgesehen davon, dass diese Gitter optisch unschön aussehen und selbst gefährlich heiß werden, können als Strahler nur solche eingesetzt werden, welche maximal sehr wenig in dem relativ gefährlichen nahen IR-Spektrum strahlen (sofern die Strahler im Sichtbereich von Menschen eingesetzt werden), da das Schutzgitter gegen dieses Spektrum keinen Schutz bietet. Da in der Strahlungsquelle das Vermeiden des besagten gefährlichen Strahlenspektrums nicht ausreichend gut selektiv gelingt, werden zwangsweise auch Strahlungsspektren vermieden, welche an sich wünschenswert wären.

In der AT 12 731 U1 wird ein Infrarotstrahler für die Beheizung einer Wellnesseinrichtung beschrieben, welcher in einem Gehäuse eine sehr heiße Infrarotstrahlungsquelle aufweist, welche an der Abstrahlungsseite des Gehäuses durch eine eher großflächige opake Glaskeramikscheibe abgedeckt ist. Hauptzweck der Glaskeramikscheibe ist es, die Strahlungsintensität von Infrarotstrahlung des nahen IR-Spektralbereichs so sehr zu vermindern, dass sie für die Augen von auf den Infrarotstrahler blickenden Menschen keine Gefahr mehr darstellt. Die Strahlungsintensität (also die Strahlungsleistung pro Raumwinkel) wird einerseits durch Großflächigkeit und Opazität der Scheibe vermindert, andererseits auch dadurch, dass das Material der Scheibe so ausgelegt sein kann, dass es einen großen Anteil der Infrarotstrahlung des potentiell gefährlichen Spektralbereiches absorbiert. Durch das Absorbieren der Strahlung und auch durch die räumliche Nähe der Glaskeramikscheibe zur heißen Infrarotstrahlungsquelle wird die Glaskeramikscheibe erhitzt. Dieses Erhitzen ist zumindest deswegen störend, weil sich dadurch Menschen, welche die Scheibe berühren, schmerzhaft verbrennen können.

Von diesem Stand der Technik ausgehend besteht die der Erfindung zu Grunde liegende Aufgabe darin, einen als Beheizung einer Wellnesseinrichtung verwendbaren Infrarotstrahler bereitzustellen, welcher neben seiner allgemeinen Heizfunktion, Strahlung in einer für die Wellnesseinrichtung vorteilhaften Spektralmischung aus Infrarotstrahlung und sichtbarer Strahlung abgibt, wobei Infrarotlicht im nahen Infrarotbereich maximal in einem ungefährlichen Ausmaß abgegeben werden darf. Es soll zwar möglich sein, dass an der Abstrahlungsseite des Infrarotstrahlers eine hohe Strahlungsleistung pro Oberfläche abgegeben wird, aber dennoch soll das Material des Infrarotstrahlers an der Abstrahlungsfläche nicht so heiß werden, wie es bei vorbekannten Infrarotstrahlern hoher Strahlungsleistung an der Abstrahlungsfläche wird.

Zum Lösen der Aufgabe wird vorgeschlagen, die Infrarotstrahlungsquelle des Infrarotstrahlers zu Abstrahlungsseite hin durch zwei in einem Abstand zueinander hintereinander angeordnete lichtdurchlässige Scheiben abzudecken, wobei die der Strahlungsquelle näher liegende Scheibe Infrarotstrahlung des potentiell gefährlichen Spektralbereichs in spürbarem Ausmaß absorbiert und wobei die von der Infrarotstrahlungsquelle weiter entfernt liegende Scheibe möglichst wenig Strahlung absorbiert.

Gegenüber einem Infrarotstrahler bei welchem eine einzige Abdeckscheibe außen durch ein Schutzgitter abgedeckt ist, wird an Vorteilen erreicht, dass der Infrarotstrahler schöner weil ebenmäßiger aussieht (damit auch besser zu reinigen ist) und dass die den Menschen zugewandten Teile weniger heiß werden. Auf Grund seiner Durchlässigkeit für Strahlung wird das Glas der äußeren Scheibe nämlich deutlich weniger durch Strahlung erhitzt als es ein stattdessen dort angebrachtes Gitter werden würde, welches de facto zwangsweise aus lichtundurchlässigem, und damit Strahlung absorbierendem Material bestehen müsste.

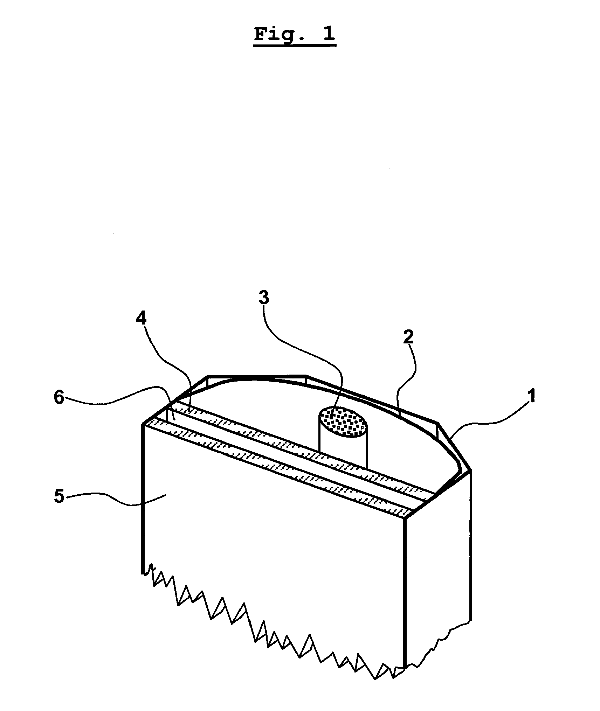

- Fig. 1:: zeigt stark stilisiert für das Verständnis der Erfindung wesentliche Teile eines beispielhaften erfindungsgemäßen Infrarotstrahlers in einer Schrägriss-Schnittansicht.

In Fig. 1 sind von einem beispielhaften erfindungsgemäßen Infrarotstrahler ein Gehäuse 1, ein darin angeordneter Reflektor 2, eine darin angeordnete Infrarotstrahlungsquelle 3 und zwei Abdeckscheiben 4, 5 dargestellt.

Alle Teile sind zu einer gemeinsamen vertikalen Symmetrieebene symmetrisch ausgebildet und angeordnet und sind in vertikaler Richtung länglich ausgebildet.

Das typischerweise aus Stahlblech bestehende Gehäuse 1 hat die Form eines aus ebenen Teilflächen gebildeten Wannenprofils. Der Reflektor 2 hat die Form eines Wannenprofils mit etwa parabelförmiger Profilquerschnittsfläche. Die Infrarotstrahlungsquelle 3 hat die Form eines schlanken kreiszylinderförmigen Stabes.

Zur bestimmungsgemäßen Abstrahlungsseite des Gehäuses 1 hin ist das Gehäuse 1 durch zwei Abdeckscheiben 4, 5 verschlossen, welche parallel zueinander und in einem Abstand zueinander angeordnet sind.

Von erfindungsgemäßer Bedeutung ist, dass die innere, also der Infrarotstrahlungsquelle 3 näher liegende Scheibe 4, besser Strahlung absorbiert als die äußere Scheibe 5. Das Absorbieren von Strahlung ist eine Form der Aufnahme von Wärmeenergie, das heißt die Scheibe 4 wird dadurch erhitzt. Die äußere Scheibe 5 ist bei bestimmungsgemäßem Betrieb des Infrarotstrahlers Menschen zugewandt angeordnet und durch diese berührbar. Indem diese äußere Scheibe 5, möglichst durchlässig - im Idealfall vollständig durchlässig - für alle Spektren von Strahlung, insbesondere für Infrarotstrahlung ist und indem ein Teil der von der Infrarotstrahlungsquelle 3 ausgesandten Strahlungsenergie schon durch die Scheibe 4 absorbiert wurde, wird die Scheibe 5 durch Strahlung kaum - im Idealfall gar nicht - erhitzt.

An Glassorten für die Scheibe 5 sind vorwiegend jene von den bekannten und kostengünstig erhältlichen Glassorten vorzusehen, die im sichtbaren und im infraroten Spektralbereich möglichst wenig absorbieren.

Natürlich kann die äußere Scheibe 5 auch so ausgeführt sein, dass sie einzelne unerwünschte Spektralbereiche des auf sie treffenden Lichtes herausfiltert. Bei der Auslegung des Filterbereichs ist aber zu beachten, dass dann, wenn ein zu hoher Anteil des Spektrums weggefiltert werden soll, störend starke Erwärmung der Scheibe auftreten kann.

Von erfindungsgemäßer Bedeutung ist weiters, dass die beiden Scheiben 4, 5 nicht aneinander anliegen, sondern dass ein leerer Zwischenraum 6 zwischen diesen beiden Scheiben liegt. Durch den Abstand zwischen den beiden Scheiben 4, 5 wird direkte Wärmeleitung von der heißen Scheibe 4 auf die kühl zu haltende Scheibe 5 unterbunden.

In einer vorteilhaften Ausführungsform ist der zwischen den beiden Scheiben befindliche Zwischenraum 6 zu zwei gegenüberliegenden Seiten hin offen, bevorzugt nach oben und nach unten hin. Damit wird ermöglicht, dass erheblich Wärme aus diesem Zwischenraum 6 durch Luftkonvektion abgeführt wird.

In einer vorteilhaften Ausführungsform ist die Strahlungsquelle 3 eine solche, welche am stärksten im Spektralbereich des nahen Infrarot (auch "Infrarot A" genannt, Wellenlängen von 0,78 bis 1,4 µm) sendet und die innere Scheibe 4 ist durch eine opak lichtdurchlässige Scheibe aus Glaskeramik gebildet. Damit lässt sich eine schöne, rasch und stark angenehm spürbare Strahlungswirkung bestmöglich mit der Vermeidung von gefährlichen Spektralanteilen kombinieren. Als Strahlungsquelle 3 kann dazu typischerweise eine Infrarot-Halogen-Lampe verwendet werden.

Glaskeramik hat einen sehr niedrigen Wärmeausdehnungskoeffizienten - womit die Gefahr von Zerstörung durch Wärmespannungen hintangehalten wird - und sie kann bekanntermaßen problemlos so zusammengesetzt sein, dass die Transmission für elektromagnetische Strahlung im Wellenlängenbereich von 0,78 bis 1,4 µm gemittelt weniger als 20% beträgt, was für den vorliegenden Zweck sehr gut ist.

## Patentansprüche

1. Infrarotstrahler für die Beheizung einer beheizten Wellnesseinrichtung, welcher eine in einem Gehäuse (1) angeordnete Infrarotstrahlungsquelle (3) aufweist sowie eine Abdeckung aus lichtdurchlässigem Material, welche sich zwischen der Infrarotstrahlungsquelle (3) und dem durch den Infrarotstrahler zu bestrahlenden Raum erstreckt,
**dadurch gekennzeichnet, dass**
die Abdeckung durch zwei Scheiben (4, 5) gebildet ist, welche bezüglich der Strahlungsrichtung in einem Abstand zueinander hintereinander angeordnet sind, wobei die beiden Scheiben (4, 5) aus zueinander unterschiedlichen Materialien bestehen, wobei das Material der der Infrarotstrahlungsquelle (3) näher liegenden Scheibe (4) einen höherem Absorptionsgrad für die von der Infrarotstrahlungsquelle (3) ausgesandte Strahlung aufweist als das Material der von der Infrarotstrahlungsquelle (3) weiter entfernt liegenden Scheibe (5).

2. Infrarotstrahler nach Anspruch 1, **dadurch gekennzeichnet, dass** die Scheibe (4) eine Glaskeramikfläche ist, deren Transmission für elektromagnetische Strahlung im Wellenlängenbereich von 0,78 bis 1,4 µm gemittelt weniger als 20% beträgt.

3. Infrarotstrahler nach Anspruch 1 oder 2, **dadurch gekennzeichnet, dass** die Infrarotstrahlungsquelle (3) am stärksten im Spektralbereich von 0,78 bis 1,4 µm Wellenlänge sendet.

4. Infrarotstrahler nach einem der Ansprüche 1 bis 3, **dadurch gekennzeichnet, dass** der Zwischenraum (6), welcher zwischen den beiden Scheiben (4, 5) liegt, an zwei gegenüberliegenden Seiten offen ist.

5. Beheizte Wellnesseinrichtung, **dadurch gekennzeichnet, dass** sie mindestens einen Infrarotstrahler nach einem der Ansprüche 1 bis 4 enthält.

6. Beheizte Wellnesseinrichtung nach Anspruch 5 und Anspruch 4, **dadurch gekennzeichnet, dass** der Infrarotstrahler so angeordnet ist, dass die Ebenen der Scheiben (4, 5) geneigt oder vertikal ausgerichtet sind und dass der Zwischenraum (6) nach oben und nach unten hin offen ist.
